# FASCICULE DE BREVET EUROPEEN

(11) **EP 3 383 403 B1**
(45) Date de publication et mention de la délivrance du brevet: **25.12.2019**
(21) Numéro de dépôt: 16809676.6
(22) Date de dépôt: 30.11.2016
(51) Int. Cl.: A61K 31/662, A61P 27/02, A61K 9/08, A61K 9/00

(54) **DÉRIVÉS AMINOPHOSPHINIQUES POUR LA PRÉVENTION ET LE TRAITEMENT DES DOULEURS OCULAIRES**
AMINOPHOSPHINDERIVATE ZUR VORBEUGUNG UND BEHANDLUNG VON AUGENSCHMERZEN
AMINOPHOSPHINIC DERIVATIVES FOR PREVENTING AND TREATING EYE PAIN

(30) Priorité: 30.11.2015 FR 1561598
(43) Date de publication de la demande: 10.10.2018
(73) Titulaire: Pharmaleads, 75013 Paris (FR)
(72) Inventeur: PORAS, Hervé, 78450 Villepreux (FR); WURM, Michel, 63130 Royat (FR); MELIK PARSADANIANTZ, Stéphane, 94130 Nogent sur Marne (FR); REAUX-LE GOAZIGO, Annabelle, 94700 Maison Alfort (FR)
(74) Mandataire: Regimbeau
(86) Numéro de dépôt international: PCT/EP2016/079285
(87) Numéro de publication internationale: WO 2017/093322

(56) Documents cités:
- ROQUES ET AL.: "INHIBITING THE BREAKDOWN OF ENDOGENOUS OPIOIDS AND CANNABINOIDS TO ALLEVIATE PAIN", NATURE REVIEW DRUG DISCOVERY, vol. 11, avril 2012 (2012-04), pages 292-310, XP002759357,
- ANONYMOUS: "PHARMALEADS ANNOUNCES PROMISING PHASE I RESULTS OF PL-37", , 29 novembre 2011 (2011-11-29), XP002759358, Extrait de l'Internet: URL:https://integrity.thomson-pharma.com/i ntegrity/xmlxsl/pk_ref_list.xml_related_re f_to?p_id=753516&p_origen=PRO&p_tsearch=#l ink [extrait le 2016-07-28]

## Description

La présente invention se rapporte à la prévention et au traitement des douleurs oculaires.

La douleur est une réponse nociceptive liée à des stimulations locales dans le corps. La perception de la douleur au niveau du système nerveux central nécessite la transmission de stimuli douloureux par les fibres nerveuses périphériques. Lors d'une stimulation au niveau des tissus, qu'elle soit thermique, mécanique ou chimique, des signaux électrochimiques sont transmis depuis les terminaisons sensorielles nerveuses vers la colonne vertébrale, et de là vers le cerveau où la douleur est ressentie.

Il existe différents types de douleur, d'origines très variées dont le traitement est radicalement différent selon le type de douleur et son étiologie.

La douleur oculaire est une forme particulière de douleur qui apparaît dans de nombreuses conditions, comme par exemple un traumatisme accidentel, une intervention chirurgicale, une uvéite, le syndrome de l'œil sec ou bien encore lors de neuropathies dues au diabète.

Les médicaments couramment utilisés pour traiter les douleurs oculaires sont les anti-inflammatoires non stéroïdiens, par voie topique ou générale, les analgésiques par voie générale, les anesthésiques locaux, ou, dans les cas extrêmes, les opiacés par voie générale. Ces traitements présentent une efficacité limitée et des effets secondaires locaux systémiques. Les anesthésiques locaux agissent sur la douleur en inhibant directement la transmission nerveuse. Leur usage est limité à une administration brève sous contrôle médical car leur mécanisme d'action induit des effets inhibiteurs au niveau cellulaire dans les fibroblastes ou bien encore les cellules neuronales environnantes. C'est pourquoi, même si la sensation de douleur peut être atténuée par les anesthésiques locaux, la cicatrisation et la physiologie des tissus peuvent être altérés. Il est donc important de découvrir d'autres agents analgésiques, sans activité anesthésique, efficaces et bien tolérés lorsqu'ils sont appliqués en topique dans l'œil douloureux.

Les opiacés comme le sulfate de morphine, sont utilisés par voie générale pour le traitement de la douleur oculaire sévère, mais ils présentent de nombreux effets indésirables comme la sédation, les nausées, la constipation, la dépression respiratoire, qui limitent considérablement leur utilisation notamment dans les douleurs oculaires chroniques.

Il a été démontré que l'administration topique de morphine soulageait les douleurs associées aux lésions de la cornée chez le chien (Stiles et al. (2003) Am. J. Vet. Res., 64, 813-818) et le rat (Wenk et al. (2003) Pain, 105, 455-465) sans causer de retard de cicatrisation de la plaie de la cornée (Stiles et al. (2003) Am. J. Vet. Res., 64, 813-818).

La perception, la transmission et la régulation des influx nociceptifs sont sous la dépendance de plusieurs neurotransmetteurs, et en particulier les enképhalines (Met-enképhaline et Leu-enképhaline). Celles-ci sont des pentapeptides, opioïdes endogènes, initialement trouvés dans le cerveau de mammifères (Hugues et al. (1975) Nature, 258, 577-580). Elles se lient principalement à deux classes de récepteurs, les récepteurs opioïdes µ et δ (Lord et al. (1977) Nature, 267, 495-499) dont les fonctions et les localisations sont différentes (Waksman et al. (1986) Proc. Natl. Acad. Sci., 83, 1523-1527).

Les propriétés antinociceptives des enképhalines ont été démontrées après administration par voie intracérébroventriculaire d'enképhalines exogènes (Belluzi et al. (1976) Nature, 260, 625-626). Cependant cette réponse est très fugace à cause d'une métabolisation très rapide de ces peptides par des enzymes. Des analogues d'enképhalines synthétiques, modifiés pour les rendre résistants à la dégradation enzymatique ont montré des propriétés antinociceptives égales à celles de la morphine, mais ont également présenté les mêmes effets secondaires indésirables que la morphine.

D'autre part, on sait que les enképhalines (Tyr-Gly-Gly-Phe-Met et Tyr-Gly-Gly-Phe-Leu) sont physiologiquement inactivées par deux métallopeptidases à zinc, la néprilysine (EC 3.4.24.11, NEP) qui clive la liaison Gly³-Phe⁴ (Malfroy et al. (1978) Nature, 276, 523-526) et l'aminopeptidase N (EC 3.4.11.2, APN) qui coupe la liaison Tyr¹-Gly² de ces peptides. (Waksman et al. (1985) Eur. J. Pharmacol., 117, 233-243; revue dans Roques et al. (1993) Pharmacol. Rev., 45, 87-146).

L'inhibition de ces deux activités enzymatiques, en protégeant complètement les enképhalines (Bourgoin et al. (1986) J. Pharm. Exp. Ther., 238, 360-366), révèlent les activités pharmacologiques et en particulier analgésiques et antidépressives (Roques (2000) Trends Pharmacol. Sci., 21, 475-483; Jutkiewicz et al. (2007) CNS Drugs Reviews, 13, 192-205) des opioïdes endogènes, les enképhalines.

Des dérivés aminophosphiniques, « vrais » inhibiteurs mixtes, c'est-à-dire inhibant conjointement l'APN et la NEP, ont été décrit dans de précédents brevets et publications (WO9818803; WO2010010106; Chen et al. (2000) J. Med. Chem., 43, 1398-1408; Chen et al. (2001) J. Med. Chem., 44, 3523-3530; Le Guen et al. (2003) Pain, 104, 139-148; Bonnard et al. (2015) Pharmacol. Res. Persp., 3(2), e00116, doi: 10.1002/prp2.116).

Une bonne activité antinociceptive, avec une longue durée d'action, a été démontrée sur de nombreux modèles animaux de nociception après administration orale et/ou iv (Chen et al. (2000) J. Med. Chem., 43, 1398-1408; Chen et al. (2001) J. Med. Chem., 44, 3523-3530; Le Guen et al. (2003) Pain, 104, 139-148; Bonnard et al. (2015) Pharmacol. Res. Persp., 3(2), e00116, doi: 10.1002/prp2.116).

L'utilisation de ces composés comme analgésiques topiques oculaires n'a jamais été décrite ou suggérée.

Il n'a également jamais été démontré que des prodrogues d'inhibiteurs mixtes de NEP et d'APN pouvaient s'hydrolyser pour donner un composé capable d'atteindre sa cible permettant ainsi une réponse analgésique de longue durée.

L'un des objets de cette invention est donc de fournir de nouveaux composés de type aminophosphiniques capables d'inhiber conjointement les deux activités enzymatiques (néprilysine et aminopeptidase N) responsables de la dégradation des enképhalines, ces derniers pouvant avoir une action dans le cadre de douleurs oculaires.

L'invention a plus particulièrement pour objet des composés répondant à la formule (I) :

(I) R₁-NH-CH(R₂)-P(=O)(OH)-CH₂-C(R₃)(R₄)-CONH-C(R₅)(R₆)-COOR₇

Dans laquelle :
R₁ représente
   - un hydrogène
   - un groupement (acyloxy)alkyl carbamate -C(=O)-O-C(R)(R')-OC(=O)-R" dans lequel R et R' représentent, indépendamment l'un de l'autre, un hydrogène, un groupement alkyle et R" représente un groupement alkyle.
R₂ représente :
   - une chaîne hydrocarbonée, saturée ou insaturée, linéaire ou ramifiée comportant de 1 à 6 atomes de carbone
R₃ et R₄ représentent indépendamment l'un de l'autre:
   - un hydrogène
   - un groupement phényle ou benzyle, éventuellement substitué sur le noyau phényle par :
      * 1 à 5 atomes d'halogènes notamment le fluor ou le brome.
      * un radical OH, SH, OR" ou SR", R" ayant la même définition que précédemment.
      * un groupement amino éventuellement mono- ou di-substitué par un groupement aliphatique, cyclique ou linéaire, de 1 à 6 atomes de carbone.
      * un groupement trifluorométhyle
      * un groupement aromatique ou hétéroaromatique à 5 ou 6 atomes
   - un groupement hétéroaromatique à 5 ou 6 atomes, contenant 1 ou 2 hétéroatome(s) pris parmi l'oxygène, l'azote ou le soufre, les atomes de soufre et d'azote pouvant être oxydés sous forme de S-oxyde ou de N-oxyde.
   - un méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme N-oxyde ou de S-oxyde R₃ et R₄ ne représentent pas simultanément un atome d'hydrogène.
R₅ et R₆ représentent indépendamment l'un de l'autre
   - un atome d'hydrogène
   - une chaîne hydrocarbonée saturée ou insaturée, linéaire ou ramifiée de 1 à 6 atomes de carbone
R₇ représente
   - un hydrogène
   - un radical CH₂COOR'" ou CH(CH₃)COOR"', R'" représentant
      *une chaîne hydrocarbonée saturée de 1 à 6 atomes de carbone, éventuellement substituée par un groupement alkoxy de C₁ à C₃,
      *un groupement cycloalkyle de C₅ à C₈
      *un groupement phényle, benzyle, hétéroaromatique ou alkyl hétéroaromatique.
   - un groupement CH(R)O-C(O)OR' ou CH(R)OC(O)R' dans lesquels R et R' ont les mêmes définitions que précédemment;
ou un sel pharmaceutiquement acceptable de ces composés pour leur utilisation dans le traitement et/ou la prévention des douleurs oculaires.

Les composés selon l'invention peuvent avantageusement être utilisés pour prévenir ou soulager une douleur oculaire, en particulier le syndrome de l'œil sec.

Les composés selon l'invention peuvent avantageusement être utilisés pour prévenir ou traiter les douleurs neuropathiques de la cornée, en particulier l'hyperalgésie neuropathique.

### Douleur oculaire

Les douleurs neuropathiques présentent des causes périphériques, centrales et peuvent spécifiquement affecter la cornée de l'œil. Les causes des neuropathies sont nombreuses, mais trouvent toujours leur origine dans la souffrance ou la détérioration de fibres nerveuses, dues à des incisions chirurgicales, un traumatisme, des virus (zona), des troubles métaboliques (diabète), des médicaments neurotoxiques ou une inflammation chronique. Les médicaments actuels sont peu efficaces, ce qui rend indispensable la découverte et le développement de nouveaux composés.

L'hyperalgésie neuropathique de la cornée relève d'un dysfonctionnement du système de perception et de contrôle de la douleur au niveau de la cornée. Elle s'associe à une gêne fonctionnelle marquée et à une sensibilité persistante accrue de la cornée (sensibilisation périphérique) en l'absence de traumatisme manifeste ou de stimuli nocifs (revue dans Belmonte et al. (2004) Exp. Eye Res., 78(3), 513-525; Rosenthal & Borsook (2012) Ocul. Surf., 10(1), 2-14; Rosenthal et al. (2009) Ocul. Surf., 7(1), 28-40).

L'excitation continue des nerfs de la cornée à la suite de lésions ou de l'irritation de celle-ci libère des neuropeptides et des médiateurs de l'inflammation qui augmentent la réaction inflammatoire (inflammation neurogène) conduisant ainsi à l'hyperalgésie. L'hypersensibilité cornéenne, la neuro-inflammation, la douleur et la photophobie sont rapportées chez des patients après chirurgie de l'œil ou après une exposition chimique ou toxique, y compris l'utilisation répétée de chlorure de benzalkonium, conservateur habituel des collyres. La douleur neuropathique cornéenne est également une conséquence de troubles oculaires, désignée de manière générale comme syndrome de l'œil sec. Cela comprend les causes immunologiques non infectieuses telles que le syndrome de Goujerot-Sjögren ou le lupus systémique ainsi que le zona ophtalmique du à *Herpesvirus zoster* (revue dans Rosenthal & Borsook (2012) Ocul. Surf., 10(1), 2-14; Yawn et al. (2013) Mayo Clin. Proc., 88(6), 562-570). Jusqu'à 20% des adultes âgés de 45 ans ou plus sont touchés par le syndrome de l'œil sec, ce qui représente un problème de santé majeur avec des conséquences économiques et sociales lourdes (revue dans Friedman (2010) Curr. Opin. Ophthalmol., 21(4), 310-316; Pfugfelder (2008) J. Manag. Care, 14 (3 Suppl), S102-S106).

Dans de très nombreux cas, le syndrome de l'œil sec est réfractaire au traitement et il n'existe pas de réelle corrélation entre symptômes et signes associés. Par exemple, alors que l'hyperalgésie de la cornée inflammatoire, en raison de la dessiccation de la surface oculaire (évaporation de l'œil sec), est la forme la plus commune de l'hyperalgésie neuropathique de la cornée, de nombreux patients présentant des symptômes de sécheresse oculaire n'ont pas de signes objectifs d'oeil sec (réduction du volume lacrymal), ou d'érosions cornéennes superficielles. En outre, la neuropathie peut parfois précéder des altérations du film lacrymal (Rosenthal & Borsook (2012) Ocul. Surf., 10(1), 2-14; Rosenthal et al. (2009), Ocul. Surf., 7(1), 28-40).

Les composés prescrits pour les douleurs neuropathiques de la cornée comprennent une grande variété de composés distincts tels que les opiacés, les anti-inflammatoires non-stéroïdiens, les inhibiteurs des canaux sodiques (anesthésiques locaux), les antiépileptiques, les antidépresseurs tricycliques et les analogues de GABA. Cet arsenal reste cependant insuffisant et la nature complexe de la douleur cornéenne neuropathique est mise en évidence par le fait qu'il n'existe pas de traitement connu unique efficace sur les différents symptômes. De plus, les effets secondaires de nombreux agents actuellement prescrits limitent la fenêtre thérapeutique. La douleur neuropathique inflammatoire de la cornée représente donc un besoin thérapeutique non satisfait (Rosenthal & Borsook (2012) Ocul. Surf., 10(1), 2-14; Rosenthal et al. (2009) Ocul. Surf., 7(1), 28-40).

### Composés selon l'invention :

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent.

Dans le cadre de la présente invention, il s'agit de sels d'addition obtenus avec une base minérale ou organique. Ainsi, le sel formé correspond :
- soit au remplacement d'un proton acide par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium,
- soit à la coordination de ce proton acide avec une base organique ou inorganique.

Les bases organiques acceptables comprennent des amines telles que l'ammoniaque, la diéthanolamine, l'éthanolamine, la N-méthylglucamine, la triéthanolamine, la triéthylamine, la trométhamine et similaires. Les bases inorganiques acceptables comprennent l'hydroxyde d'aluminium, l'hydroxyde de calcium, l'hydroxyde de lithium (lithine), l'hydroxyde de potassium (potasse), le carbonate de sodium et l'hydroxyde de sodium (soude).

Avantageusement, les sels pharmaceutiquement acceptables des composés de l'invention seront des sels d'addition obtenus avec une base minérale ou organique pharmaceutiquement acceptable, telle que la lithine, la soude, la potasse, l'ammoniaque, une amine tertiaire de formule NRₐR_{b}R_{c}, où Rₐ, R_{b} et R_{c} représentent, indépendamment les uns de autres, un groupe alkyle tel que défini ci-dessous, comme la triéthylamine, ou encore un aminoacide basique tel que la lysine ou l'arginine et leurs dérivés.

Par « insaturé », on entend, au sens de la présente invention, que la chaîne hydrocarbonée comprend une ou plusieurs insaturation(s). Par « insaturation », on entend, au sens de la présente invention, une double ou une triple liaison.

Par « atome d'halogène », on entend, au sens de la présente invention, un atome de fluor, de chlore, de brome ou d'iode. Avantageusement, il s'agit d'un atome de fluor, de brome ou de chlore. Encore avantageusement, il s'agit d'un atome de fluor ou de brome, et de préférence de fluor.

Par groupe « amino », on entend, au sens de la présente invention, un groupe de formule -NR*R**, où R* et R** représentent, indépendamment l'un de l'autre, un atome d'hydrogène ou un groupe hydrocarboné saturé ou insaturé, linéaire, ramifié ou cyclique, comportant de 1 à 6, de préférence de 1 à 4, atomes de carbone, ou R* et R** forment ensemble, avec l'atome d'azote qui les porte, un hétérocycle à 5 ou 6 chaînons, saturé ou non, et ne comportant pas d'autre hétéroatome que l'azote qui porte les deux radicaux R* et R**. En particulier, le groupe amino peut être un groupe -NH₂, -NHMe, -NHEt, -NHPr, NHiPr, -NHBu, -NHiBu, -NHtBu, pipéridinyle ou pyrrolidinyle.

Par groupement « aromatique », on entend, au sens de la présente invention, un groupement aromatique, comportant de préférence de 5 à 10 atomes de carbone, sauf mention contraire, et comprenant un ou plusieurs cycles accolés, comme par exemple un groupement phényle ou naphtyle. Avantageusement, il s'agit du phényle.

Par groupement « hétéroaromatique », on entend, au sens de la présente invention, tout groupe aromatique tel que défini ci-dessus dans lequel un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydé sous forme de S-oxyde ou de N-oxyde. Des exemples de groupes hétéroaromatique sont les groupes furyle, thiényle, pyrrolyle, pyridinyle, pyrimidyle, pyrazolyle, imidazolyle, tétrazolyle ou encore indyle.

Par « cycle hétéroaromatique à 5 ou 6 atomes », on entend, au sens de la présente invention, un groupe hétéroaromatique tel que défini ci-dessus ne comportant qu'un seul cycle à 5 ou 6 atomes. Il s'agit notamment d'un groupe thiényle, pyrrolyle, pyridinyle, pyrimidyle, pyrazolyle, imidazolyle ou encore tétrazolyle.

Par « hétérocycle », on entend, au sens de la présente invention, un cycle hydrocarboné, avantageusement à 5 ou 6 atomes, dont un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydés sous forme de N-oxyde et de S-oxyde. Sauf mention contraire, ce cycle pourra être saturé ou aromatique.

Dans le cas où le ou les hétéroatome(s) est (sont) choisi(s) parmi l'azote et le soufre, l'hétérocycle peut être en particulier un groupe : pipéridinyle, pyrrolidinyle, pyrrolyle, thiényle, pyrrazolyle, imidazolyle, pyridinyle, pyridazinyle, pyrimidinyle, pipérazinye, thiadiazolyle, tétrahydrothiényle ou encore thiazolyle.

Par « alkyle », on entend, au sens de la présente invention, une chaîne hydrocarbonée saturée, linéaire ou ramifiée, comportant de 1 à 4 atomes de carbone, sauf mention contraire. Il s'agit en particulier des groupes méthyle, éthyle, n-propyle, isopropyle, n-butyle, iso-butyle, sec-butyle, tert butyle, n-pentyle, n-hexyle.

Par « cycloalkyle », on entend, au sens de la présente invention, un cycle hydrocarboné saturé comportant de 5 à 8 atomes de carbone, en particulier le groupe cyclohexyle, cyclopentyle ou cycloheptyle.

Par «alkylehétéroaromatique », on entend, au sens de la présente invention, un groupe hétéroaromatique tel que défini ci-dessus lié à la molécule par l'intermédiaire d'un groupe alkyle tel que défini ci-dessus. Il s'agit en particulier d'un groupe thénylméthyle ou furylméthyle.

Dans une première variante, R₁ représente un groupement (acyloxy)alkyl carbamate - C(=O)-O-C(R)(R')-OC(=O)-R". En particulier, R₁ représente un groupement -C(=O)-O-CHMe-OC(=O)-CHMe₂.

Dans une deuxième variante, R₁ représente un atome d'hydrogène.

De manière également avantageuse, le radical R₂ représente une chaîne hydrocarbonée, saturée, linéaire ou ramifiée, comportant de 1 à 4 atomes de carbone. De préférence, le radical R₂ représente un groupe méthyle.

Selon une variante avantageuse de l'invention, R₃ représente un atome d'hydrogène et R₄ est tel que défini précédemment. Avantageusement, R₃ représente un atome d'hydrogène et R₄ représente un groupe benzyle éventuellement substitué par 1 à 5 atome(s) d'halogène tel(s) que le fluor ou le brome, un phényle ou un groupement hétéroaromatique à 5 ou 6 chaînons. En particulier, R₃ représente un atome d'hydrogène et R₄ représente un groupe benzyle substitué, en position para, par un atome d'halogène, tel qu'un atome de brome, ou par un phényle.

De manière également avantageuse, le radical R₅ représente un atome d'hydrogène.

De manière également avantageuse, le radical R₆ représente un groupement alkyle tel qu'un groupement méthyle.

De manière également avantageuse, le radical R₇ représente un atome d'hydrogène ou un benzyle.

Selon une variante avantageuse de l'invention, les radicaux ont la signification suivante:
- R₁ représente un groupe -C(=O)-O-C(R)(R')-OC(=O)-R" dans lequel R représente un atome d'hydrogène et R' et R" représentent un groupe alkyle ;
- R₂ représente un groupe alkyle,
- R₃ représente un atome d'hydrogène ;
- R₄ représente un groupe benzyle substitué en position para par un atome d'halogène (brome) ou par un phényle ;
- R₅ représente un atome d'hydrogène ;
- R₆ représente un groupe alkyle ;
- R₇ représente un atome d'hydrogène.

Selon un mode de réalisation particulier, le composé de l'invention est choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-(2-(4-Bromo-benzyl)-3-{hydroxy-[1-(1-isobutyryloxy-éthoxycarbonylamino)-éthyl]-phosphinoyl}-propionylamino)-propionique
Ester benzylique de l'acide 2-(2-Biphényl-4-ylméthyl-3-{hydroxy-[1-(amino)-éthyl]-phosphinoyl}-propionylamino)-propionique
Acide 2-(2-Biphényl-4-ylméthyl-3-{hydroxy-[1-(amino)-éthyl]-phosphinoyl}-propionylamino)-propionique
Ester éthylique de l'acide 2-(2-Biphényl-4-ylméthyl-3-{hydroxy-[1-(1amino)-éthyl]-phosphinoyl}-propionylamino)-propionique
Ester éthoxycarbonyloxy de l'acide 2-(2-Biphényl-4-ylméthyl-3-{hydroxy-[1-(amino)-éthyl]-phosphinoyl}-propionylamino)-propionique
Ester benzylique de l'acide 2-(2-(4-Bromo-benzyl)-3-{hydroxy-[1-(amino)-éthyl]-phosphinoyl}-propionylamino)-propionique
Acide 2-(2-(-(amino)-éthyl]-phosphinoyl}-propionylamino)-propionique
Ester éthylique de l'acide 2-(2-(4-Bromo-benzyl)-3-{hydroxy-[1-(amino)-éthyl]-phosphinoyl}-propionylamino)-propionique

Les composés de formule (I) ont été décrits comme inhibiteurs doubles de l'APN et de la NEP et présentent des activités analgésiques dans de nombreux modèles de douleurs centrales ou périphériques après une administration intraveineuse ou orale (Chen et al. (2000) J. Med. Chem., 43, 1398-1408; Bonnard et al. (2015) Pharmacol. Res. Persp., 3(2), e00116, doi: 10.1002/prp2.116).

Les composés de formule (I) peuvent être synthétisés, par exemple, par des méthodes décrites dans: FR 2 755 135 et FR 2 934 267 . Le composé 1, par exemple, peut être synthétisé comme décrit dans Chen et al. (2000) J. Med. Chem., 43, 1398-1408 et le composé 2, par exemple, peut être synthétisé comme décrit dans Bonnard et al. (2015) Pharmacol. Res. Persp., 3(2), e00116, doi: 10.1002/prp2.116.

Les composés de formule (I) sont formulés en accord avec des méthodes décrites par l'homme de l'art, en particulier pour la voie d'administration désirée.

En particulier, les composés sont formulés sous la forme d'une composition ophtalmique, en particulier de collyres, pommades ophtalmiques, gels ophtalmiques, ou inserts ophtalmiques

De manière préférentielle, les compositions administrées de la présente invention seront formulées comme solutions, suspensions ou autre dosage pour administration topique, en particulier une administration oculaire. Par conséquent, de telles compositions sont formulées de sorte à respecter : une bonne tolérance (en particulier pH acceptable), une osmolarité physiologique. Il est en outre préférable que les compositions soient stériles et formulées de sorte à éviter une contamination bactérienne en cours d'utilisation. Les solutions ophtalmiques sont avantageusement limpides, caractérisées par une absence de particules. Les suspensions sont avantageusement caractérisées par une taille des particules inférieure à 25 µm. A titre de formulations galéniques pour administration oculaire, on peut notamment citer :
- Les collyres : sous la forme de solutions, suspensions, émulsions stériles, contenant un ou plusieurs principes actifs. On préfère les solvants ou mélange de solvants aqueux ou hydrosolubles. Les collyres peuvent être conditionnés en emballage multidoses ou unidoses.
- Les pommades ophtalmiques, c'est-à-dire des préparations semi-solides, stériles, destinées à être appliquées sur la conjonctive, contenant un ou plusieurs principes actifs et des excipients appropriés (vaseline, paraffine liquide).
- Les gels ophtalmiques, c'est-à-dire des préparations semi-solides, stériles, destinées à être appliquées sur la conjonctive, contenant un ou plusieurs principes actifs et des excipients appropriés. L'excipient est avantageusement un polymère hydrophile gélifiant en présence d'eau (carbomère, carbopol®, acide polyacrylique).
- Les Inserts ophtalmiques, c'est-à-dire des préparations solides ou semi-solides stériles, destinées à être insérées dans le sac conjonctival. Ils sont en général constitués d'un réservoir de principe actif encastré dans une matrice entourée d'une membrane permettant de contrôler la libération. Le principe actif est libéré progressivement.

Les solutions aqueuses seront utilisées de préférence car elles sont plus facilement formulées, et il est également plus facile pour un patient de s'administrer une telle composition à l'aide d'une instillation de 1 ou 2 gouttes de la solution dans l'œil affecté. Malgré tout, la composition pourra également être une suspension, un gel visqueux ou semi-visqueux ou d'autres types de compositions solides ou semi-solides.

Le véhicule utilisé de préférence pour les formulations ophtalmiques de la présente invention est l'eau milliQ, et de manière préférentielle une solution saline physiologique. Afin d'éviter toute dérive du pH pendant le stockage, le pH d'une telle solution sera de préférence maintenu entre 5,5 et 8, et de manière préférentielle entre 6,5 et 7,2, avec le tampon approprié tels que des tampons acétates, citrates, phosphates ou borates. Les formulations pourront également contenir les conventionnels, acceptable pharmaceutiquement, conservateurs, stabilisants et/ou composés favorisant la pénétration.

Ainsi, la composition ophtalmique est avantageusement une solution aqueuse, ayant avantageusement un pH allant de 5,5 à 8.

Les compositions administrées en accord avec les méthodes décrites dans la présente invention contiennent une quantité active pour une utilisation ophtalmique d'un composé de formule (I). Cela signifie une quantité suffisante pour prévenir ou soulager une douleur oculaire. Généralement, les compositions décrites dans la présente invention contiendront de 0,01% à 3% (poids/volume) d'un composé de formule (I). De préférence, les compositions de la présente invention contiendront de 0,1 à 1% (poids/volume) d'un composé de formule (I). Ainsi, la composition ophtalmique comprend avantageusement de 0,01% à 3% en poids par volume dudit composé de formule (I), plus avantageusement de 0,1% à 1% en poids par volume.

La composition administrée pourra également contenir d'autres ingrédients variés tels que, mais non de manière exhaustive, des surfactants, des agents influant sur l'osmolarité, des tampons, des conservateurs, des co-solvants ou des agents augmentant la viscosité.

Différents composés influençant l'osmolarité peuvent être utilisés pour ajuster l'osmolarité d'une solution afin de se rapprocher de la composition des larmes naturelles. Par exemple, le chlorure de sodium, le chlorure de potassium, le chlorure de magnésium, le chlorure de calcium, le dextrose et/ou le mannitol peuvent être utilisés afin de se rapprocher de l'osmolarité physiologique (de manière générale de l'ordre de 150-450 mOsm et de manière préférentielle 250-350 mOsm).

Les agents conservateurs qui peuvent être utilisés dans les formulations ophtalmiques de la présente invention peuvent être, mais ne sont pas limités à, du chlorure de benzalconium, du chlorobutanol, du bromure de benzododecinium, du methyl parabène, du propyl parabène, du thimerosal, de l'acétate phenylmercurique et du nitrate phenylmercurique. De tels conservateurs sont généralement utilisés à une dose de 0,001 à 1,0% poids/volume.

Les agents agissant sur la viscosité qui peuvent être utilisés dans les formulations ophtalmiques de la présente invention peuvent être, mais ne sont pas limités à, des polyols monomériques, de la povidone, de l'hydroxypropylmethyl cellulose, des poloxamers, de la carboxymethyl cellulose, des carbomères ou de l'hydroxyethylcellulose, des dextrans comme le dextran 70, des protéines solubles dans l'eau comme la gélatine ...

Les agents agissant sur la pénétration qui peuvent être utilisés sont par exemple, des solvants organiques comme le dimethylsulfoxyde ou autres sulfoxydes, la dimethylacétamide et les pyrrolidones; certains composés amides d'amines hétérocycliques, des glycols (ex propyleneglycol); des carbonates de propylène; de l'acide oléique; des amines alkylées et autres sels ammonium dérivés; des agents de surface variés anioniques, cationiques ou non-ioniques etc ...

Dans un mode de réalisation préféré, une formulation de la présente invention comprend une cyclodextrine, telle que l'hydroxypropyl béta-cyclodextrine ou bien encore la sulfobutyl ether béta-cyclodextrine, ou du sodium de polystyrène sulfonate.

Ainsi, la composition ophtalmique comprend avantageusement en outre cyclodextrine, telle que l'hydroxypropyl béta-cyclodextrine ou bien encore la sulfobutyl ether béta-cyclodextrine, ou du sodium de polystyrène sulfonate.

### Exemple activité pharmacologique

### a) Test à la capsaïcine

Afin d'étudier l'activité pharmacologique de la présente invention dans le cas des douleurs oculaires, les composés de formule (I) ont été testés sur le modèle de la douleur oculaire induite par la capsaicine.

Dans ce modèle, précédemment utilisé dans la littérature (Gonzalez et al. (1993) Invest. Ophthalmol. Vis. Sci., 34(12), 3329-3335), chez les lapins adultes, le nombre de mouvements de la paupière, le degré d'ouverture palpébrale, la réponse myotique et une vasodilatation conjonctivale évoquée par une instillation bilatérale de 30 microlitres de la capsaïcine (33 mM) ont été mesurées à des moments différents après administration d'un médicament. Pour cela les réponses à ce test dans les yeux prétraités avec différents inhibiteurs calciques (le diltiazem, le vérapamil ou la nifédipine) ont été comparées à ceux qui avaient reçu uniquement le véhicule. Par exemple, le diltiazem à des doses de 1 à 28 mM, administré 15 minutes avant l'application de la capsaïcine, a diminué de manière significative les mouvements de grattage, d'hyperémie conjonctivale, la fermeture de l'œil et la concentration élevée de protéine aqueuse induite par la capsaïcine. Ces résultats suggèrent que pour une activité douloureuse induite par la capsaïcine, le diltiazem diminue celle-ci ainsi que l'inflammation neurogène et qu'il peut être utile à la fois comme un analgésique et comme un agent anti-inflammatoire de l'œil.

Il a également été montré que les réponses contractiles à la capsaïcine étaient partiellement inhibées par la morphine (5 X 100 µM) (Zhang et al. (1984) Exp. Eye Res., 38, 153-163).

De la même manière chez le rat, l'application topique d'une goutte du sulfate de morphine (5µM) atténue le clignotement des yeux dépendamment de la concentration induite par la capsaïcine. Cependant, la morphine n'a aucun effet sur le clignotant sur la cornée de rat sain, non enflammée. Aussi bien les effets analgésiques et anti-inflammatoires de la morphine ont été bloqués par l'administration préalable d'antagonistes des récepteurs opioïdes comme la naloxone, le CTAP et le naltrindole. La morphine agit sur les récepteurs opioïdes mu et delta situés dans la cornée de rat pour atténuer l'inflammation et l'hyperalgésie (Zhang et al. (2003) Pain, 105, 455-65).

### 1) Traitement « acute »

Un test comparable à celui précédemment décrit a été utilisé (Gonzalez et al. (1993) Invest. Ophthalmol. Vis. Sci., 34(12), 3329-3335) pour évaluer les composés de formule (I) de la présente invention chez des lapins blancs albinos de Nouvelle Zélande avec comme contrôle positif un antagoniste TRPV1, la capsazepine instillée à 5mM (8 animaux par groupe) en comparaison du véhicule seul, NaCl 0,9% (3 animaux).

La procédure suivie et le timing étaient les suivants:

| **Jour** | **Temps** | **Procédure** | **Examen oculaire** |
|---|---|---|---|
| - | | Examen clinique général, poids | Evaluation de l'hyperémie conjonctive Mesure de l'ouverture palpébrale |
| D0 | 15 min avant induction | Examen clinique général Administration du produit test, contrôle et de référence (50 µL dans l'œil droit pour le produit test et le contrôle et 30µL dans l'œil droit pour le produit de référence) | Evaluation de l'hyperémie conjonctive |
| | Juste avant induction | - | Evaluation de l'hyperémie conjonctive Mesure de l'ouverture palpébrale |
| | 0 | Induction de la douleur oculaire avec capsaicine (30 µL dans l'œil droit) | - |
| | 1 min | - | Evaluation de l'hyperémie conjonctive Mesure de l'ouverture palpébrale |
| | 5 min | | |
| | 10 min | | |
| | 15 min | | |
| | 20 min | | |
| | 25 min | | |
| | 30 min | | |
| | 40 min | | |
| | 50 min | | |
| | 60 min | | |
| | - | | - |

### Exemple 1:

Effet analgésique du **composé 1 (10 mM)** dans le modèle de la douleur oculaire induit par la capsaicine (33 mM)

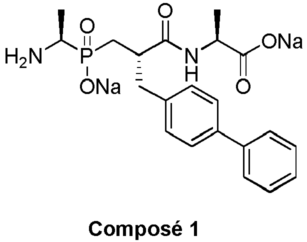

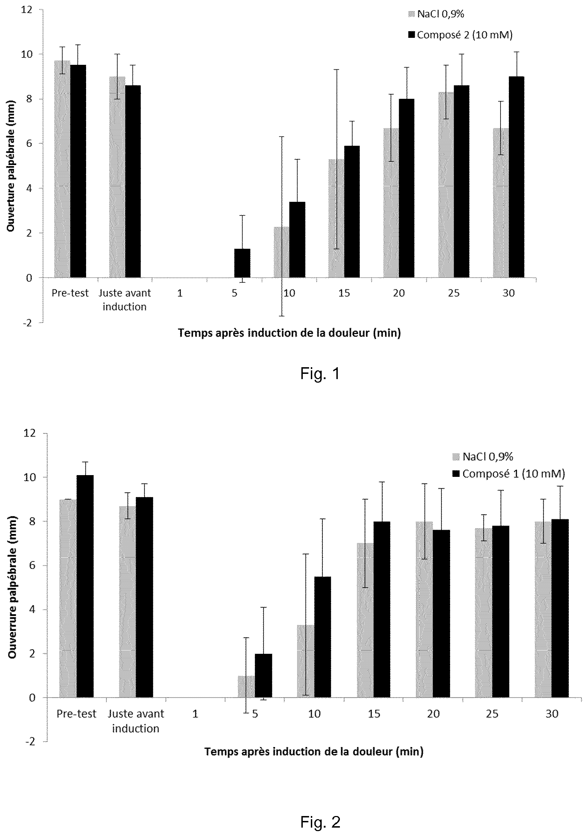

Les résultats sont reportés sur la figure 1 représentant le degré d'ouverture palpébrale, en mm, lors du pré-test, juste avant l'induction de la douleur puis à 5, 10, 15, 20, 25 et 30 min après induction de la douleur.
Histogrammes gris : la solution administrée comprend NaCl 0,9%
Histogrammes noirs : la solution administrée comprend 10 mM de composé 1

### Exemple 2:

Effet analgésique du **composé 2 (10 mM)** dans le modèle de la douleur oculaire induit par la capsaicine (33 mM)

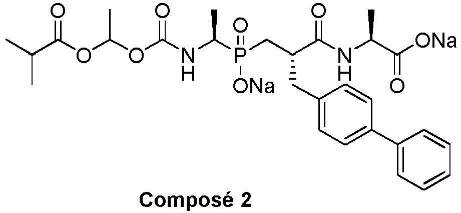

Les résultats sont reportés sur la figure 2 représentant le degré d'ouverture palpébrale, en mm, lors du pré-test, juste avant l'induction de la douleur puis à 5, 10, 15, 20, 25 et 30 min après induction de la douleur.
Histogrammes gris : la solution administrée comprend NaCl 0,9%
Histogrammes noirs : la solution administrée comprend 10 mM de composé 2

### Exemple 3:

Évaluation de la douleur de la cornée en observant l'hyperémie conjonctivale après l'induction de la douleur (une seule instillation de capsaïcine 1% (33 mM) dans l'œil droit) chez les lapins albinos. Prétraitement par **le composé à tester,** 15 min avant instillation par la capsaïcine.

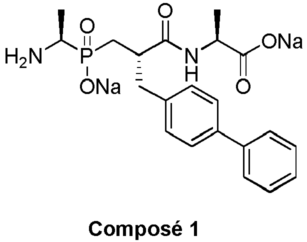

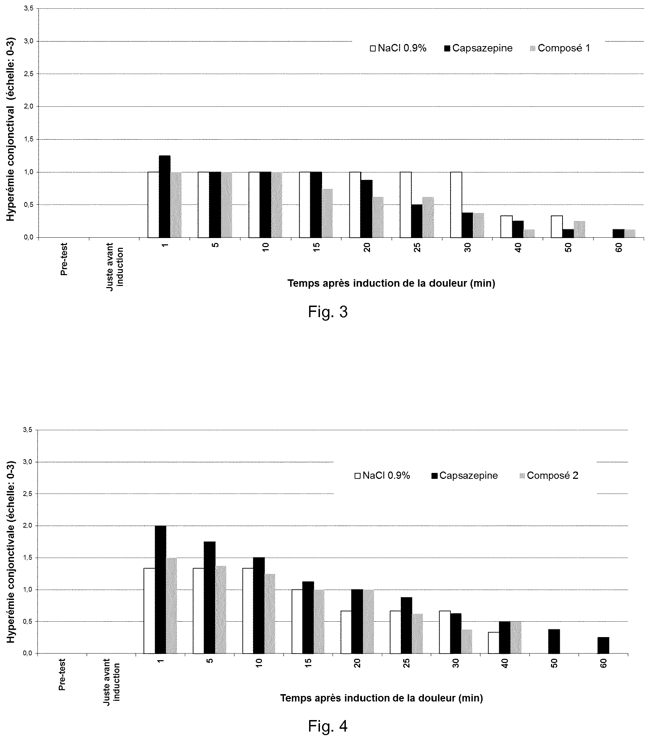

Les résultats sont reportés sur la figure 3 représentant l'hyperémie conjonctivale (échelle : 0-3) lors du pré-test, juste avant l'induction de la douleur puis à 1, 5, 10, 15, 20, 25, 30, 40, 50 et 60 min après induction de la douleur.
Histogrammes blancs : la solution administrée comprend NaCl 0,9%
Histogrammes noirs : la solution administrée comprend 5 mM de capsazépine (produit contrôle de référence)
Histogrammes gris : la solution administrée comprend 10 mM de **composé 1**

### Exemple 4:

Évaluation de la douleur de la cornée en observant l'hyperémie conjonctivale après l'induction de la douleur (une seule instillation de capsaïcine 1% (33 mM) dans l'œil droit) chez les lapins albinos. Prétraitement par **le composé à tester,** 15 min avant instillation par la capsaïcine.

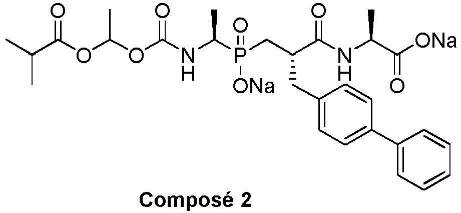

Les résultats sont reportés sur la figure 4 représentant l'hyperémie conjonctivale (échelle : 0-3) lors du pré-test, juste avant l'induction de la douleur puis à 1, 5, 10, 15, 20, 25, 30, 40, 50 et 60 min après induction de la douleur.
Histogrammes blancs : la solution administrée comprend NaCl 0,9%
Histogrammes noirs : la solution administrée comprend 5 mM de capsazépine (produit contrôle de référence)
Histogrammes gris : la solution administrée comprend 10 mM de composé 2.

Dans nos conditions expérimentales, une seule instillation du **composé 1** ou du **composé 2** à 10 mM a montré un effet analgésique suite à l'induction de la douleur par la capsaïcine, sur l'hyperémie conjonctivale et l'ouverture de la fente palpébrale, supérieure au produit de référence, la capsazépine.

### 2) Traitement « chronique »

Les composés de formule I de la présente invention ont également été testés, chez des souris mâles C57BL/6 (JANVIER LABS) âgés de 8 semaines, sur un test à la capsaicine (100 µM) après un traitement biquotidien pendant 5 jours de composé de formule I sur une cornée préalablement lésée (avec tréphine de 1,5 mm de diamètre).

### Exemple 5

Effet analgésique du **composé 2** dans le modèle de la douleur oculaire induit par la capsaicine (100 µM)

Une lésion épithéliale cornéenne a été réalisée sur la cornée droite de souris mâles C57BL/6 (JANVIER LABS) à l'aide d'une tréphine de 1,5 mm de diamètre. Une fois les animaux opérés, un traitement topique biquotidien, pendant 4 jours, est réalisé au PBS 1x (5 animaux par groupe) ou avec le **composé 2** à 10 mM (5 animaux par groupe). Au jour 4, 15 min après la dernière instillation, de la capsaicine 100 µM est instillée et l'analyse comportementale est réalisée.

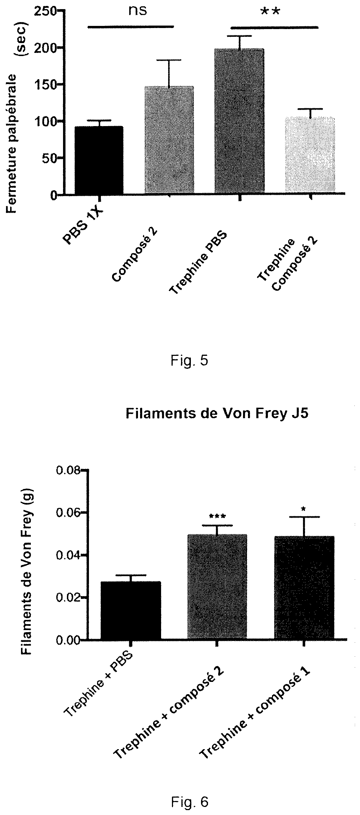

Les résultats obtenus correspondant à la lésion à la tréphine seule avant capsaicine sont représentés sur la figure 5 (axe des ordonnées : temps de fermeture palpébrale en secondes).

Les résultats obtenus après application de la capsaicine sont représentés sur la figure 6.

Dans ces conditions expérimentales, un traitement chronique par le **composé 2** à 10 mM a montré un effet analgésique très significatif (temps cumulé de fermeture palpébral plus court), par rapport au groupe contrôle recevant du PBS, suite à l'induction de la douleur dans le test à la capsaicine (100 µM).

### a) Lésion cornéenne à l'aide d'une tréphine chez la souris

Lors de ce test, la désépithélialisation de l'épithélium cornéen de l'œil gauche de la souris est réalisée à l'aide d'une tréphine de 1,5 mm de diamètre. Cette chirurgie est réalisée sous un microscope opératoire. Cet acte dure au maximum 3 minutes par animal. La profondeur et la superficie de la désépithélialisation de la cornée est standardisée et est ensuite contrôlée par un examen à la lampe à fente. L'avantage de cet outil chirurgical est la standardisation de la lésion.

Des souris mâles C57BL/6 ainsi traitées, âgées de 8 semaines, reçoivent un traitement biquotidien pendant 5 jours de composé de formule I ou de PBS avant l'application de la capsaicine.

### Exemple 6

Une douleur supplémentaire est induite par la capsaicine (100 µM).

La sensibilité (allodynie) mécanique cornéenne a été mesurée à l'aide de filaments de Von Frey, 5 jours après traitement, sur des souris traitées avec le **composé 1** (10mM), le **composé 2** (10mM) ou pour le groupe contrôle par du PBS 1X (n= 5 animaux par groupe).

Les résultats obtenus sont présentés sur la figure 6 (axe des ordonnées : poids du filament de Von Frey en g). L'histogramme de gauche (noir) représente la réponse des souris traitées avec du PBS, l'histogramme du milieu représente la réponse des souris traitées avec le **composé 1** et l'histogramme de droite représente la réponse des souris traitées avec le **composé 2.**

Un traitement topique biquotidien de 5 jours avec le **composé 1** ou le **composé 2,** sur une cornée préalablement lésée (tréphine de 1,5 mm de diamètre), induit une diminution significative, lors de la mesure avec les filaments de Von Frey, de la sensibilité mécanique (ce qui se traduit par un poids plus élevé acceptable du filament) en comparaison avec les résultats obtenus chez des souris contrôles traitées avec du PBS1x (n=5 souris par groupe).

### b) Kératite induite par instillation chronique de chlorure de benzalkonium (BAC) à 0,2%

L'instillation chronique de chlorure de benzalkonium à 0,2% induit une inflammation chronique de la cornée avec génération de douleur oculaire (Launay et al. (2016) Neurobiol. Dis., 88 16-28). Ce modèle a été utilisé comme modèle de kératite chimique.

### Exemple 7

Etude de la sensibilité cornéenne chez des souris traitées bi-quotidiennement avec le **composé 2** pendant 5 jours préalablement traitées au BAC 0,2 % pendant 6 jours

La sensibilité cornéenne mécanique des souris mâles C57BL/6, traitées au BAC 0,2 % pendant 6 jours, puis bi-quotidiennement avec le **composé 2** pendant 5 jours, a été évaluée à différents temps par les filaments de Von Frey, par rapport à un groupe contrôle recevant bi-quotidiennement du PBS 1X (n = 10 animaux par groupe).

L'utilisation des filaments de Von Frey (appliqués au centre de la cornée sur animal vigile) est tout à fait réalisable pour mesurer une allodynie mécanique cornéenne chez la souris. Dans un contexte de sècheresse oculaire, il se produit une allodynie mécanique chez le patient : le simple fait de fermer la paupière induit une sensation douloureuse. Enfin, ce test comportemental est celui qui se rapproche le plus de celui utilisé en clinique humaine (Cochet Bonnet) pour mesurer la sensibilité mécanique cornéenne.

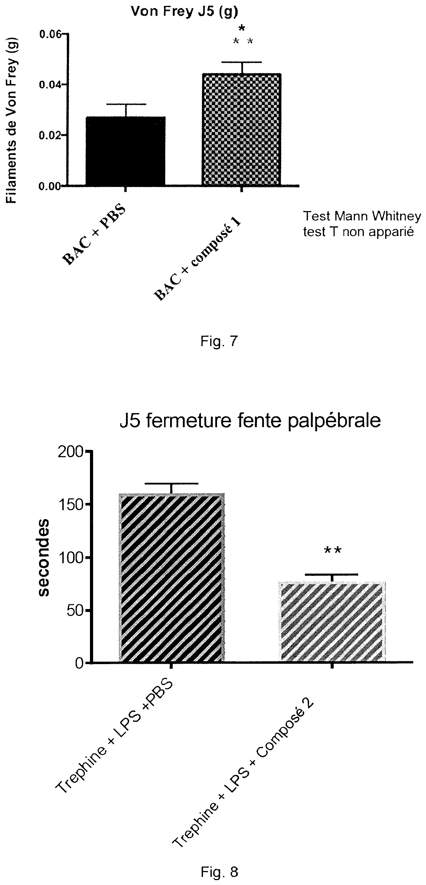

Les résultats obtenus sont présentés sur la figure 7 (axe des ordonnées : poids du filament de Von Frey en g). L'histogramme de gauche représente la réponse des souris traitées avec du PBS, tandis que l'histogramme de droite représente la réponse des souris traitées avec le **composé 2.**La figure **7** montre que l'allodynie mécanique induite par le BAC 0,2% est significativement réduite (seuil de sensibilité augmenté) en présence du **composé 2** (10 mM).

### c) Modèle inflammatoire induit par du lipopolysaccharide (LPS) chez la souris

Le lipopolysaccharide bactérien (LPS, endotoxine) est un puissant stimulateur de réponses inflammatoires qui contribue à des kératites microbiennes et aux ulcérations de la cornée (Khatri et al. 2002 Invest. Ophthal. Vis. Sci., 43 2278-2284).

### Exemple 8

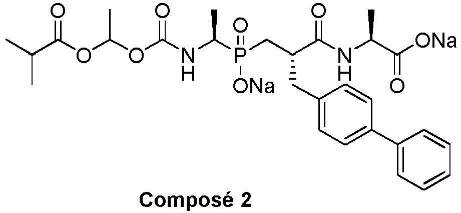

Effet du **composé 2** dans un modèle inflammatoire induit par du LPS (50 µg).

Une lésion épithéliale cornéenne (scratch) est réalisée avec une tréphine (diamètre 1,5 mm) chez la souris, suivie par un traitement topique au LPS (50 µg, deux administrations aux 1^{er} et 4^{ème} jour) puis un traitement chronique biquotidien pendant 5 jours au PBS 1X (groupe contrôle) ou avec le **composé 2** (10 mM) (n= 6 animaux par groupe), suivi par une instillation de capsaicine (100µM) au 5^{ème} jour.

Les résultats obtenus sont présentés sur la figure 8 (axe des ordonnées : temps de fermeture palpébrale en secondes). L'histogramme de gauche représente la réponse des souris traitées avec du PBS, tandis que l'histogramme de droite représente la réponse des souris traitées avec le **composé 2.**

Dans ces conditions expérimentales de kératite bactérienne induite par le LPS, un traitement chronique par le **composé 2** à 10 mM montre donc un effet analgésique très significatif (temps cumulé de fermeture de la fente palpébrale plus court), par rapport au groupe contrôle recevant du PBS.

## Revendications

1. Composés répondant à la formule (I) :
(I) R₁-NH-CH(R₂)-P(=O)(OH)-CH₂-C(R₃)(R₄)-CONH-C(R₅)(R₆)-COOR₇
Dans laquelle :
R₁ représente
- un hydrogène
- un groupement (acyloxy)alkyl carbamate -C(=O)-O-C(R)(R')-OC(=O)-R" dans lequel R et R' représentent, indépendamment l'un de l'autre, un hydrogène, un groupement alkyle et R" représente un groupement alkyle.
R₂ représente :
- une chaîne hydrocarbonée, saturée ou insaturée, linéaire ou ramifiée comportant de 1 à 6 atomes de carbone
R₃ et R₄ représentent indépendamment l'un de l'autre:
- un hydrogène
- un groupement phényle ou benzyle, éventuellement substitué sur le noyau phényle par :
* 1 à 5 atomes d'halogènes notamment le fluor ou le brome.
* un radical OH, SH, OR" ou SR", R" ayant la même définition que précédemment.
* un groupement amino éventuellement mono- ou di-substitué par un groupement aliphatique, cyclique ou linéaire, de 1 à 6 atomes de carbone.
* un groupement trifluorométhyle
* un groupement aromatique ou hétéroaromatique à 5 ou 6 atomes
- un groupement hétéroaromatique à 5 ou 6 atomes, contenant 1 ou 2 hétéroatome(s) pris parmi l'oxygène, l'azote ou le soufre, les atomes de soufre et d'azote pouvant être oxydés sous forme de S-oxyde ou de N-oxyde.
- un méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme N-oxyde ou de S-oxyde.
R₃ et R₄ ne représentent pas simultanément un atome d'hydrogène
R₅ et R₆ représentent indépendamment l'un de l'autre
- un atome d'hydrogène
- une chaîne hydrocarbonée saturée ou insaturée, linéaire ou ramifiée de 1 à 6 atomes de carbone
R₇ représente
- un hydrogène
- un radical CH₂COOR"' ou CH(CH₃)COOR"', R'" représentant
*une chaîne hydrocarbonée saturée de 1 à 6 atomes de carbone, éventuellement substituée par un groupement alkoxy de C₁ à C₃,
*un groupement cycloalkyle de C₅ à C₈
*un groupement phényle, benzyle, hétéroaromatique ou alkyl hétéroaromatique.
- un groupement CH(R)O-C(O)OR' ou CH(R)OC(O)R' dans lesquels R et R' ont les mêmes définitions que précédemment ;
ou un sel pharmaceutiquement acceptable de ces composés pour leur utilisation dans le traitement et/ou la prévention des douleurs oculaires.

2. Composé pour son utilisation selon la revendication 1, pour prévenir ou soulager une douleur oculaire, en particulier le syndrome de l'œil sec.

3. Composé pour son utilisation selon la revendication 1 ou 2, pour prévenir ou traiter les douleurs neuropathiques de la cornée, en particulier l'hyperalgésie neuropathique.

4. Composé pour son utilisation selon l'une quelconque des revendications précédentes, **caractérisé en ce que** R₁ représente un groupement (acyloxy)alkyl carbamate -C(=O)-O-C(R)(R')-OC(=O)-R".

5. Composé pour son utilisation selon l'une quelconque des revendications précédentes, **caractérisé en ce que** R₁ représente un groupement -C(=O)-O-CHMe-OC(=O)-CHMe₂.

6. Composé pour son utilisation selon l'une quelconque des revendications précédentes, **caractérisé en ce que** R₃ représente un atome d'hydrogène.

7. Composé pour son utilisation selon l'une quelconque des revendications précédentes, **caractérisé en ce que** R₄ représente groupe benzyle éventuellement substitué par 1 à 5 atome(s) d'halogène tel(s) que le fluor ou le brome, un phényle ou un groupement hétéroaromatique à 5 ou 6 chaînons.

8. Composé pour son utilisation selon l'une quelconque des revendications précédentes, **caractérisé en ce que** R₅ représente un atome d'hydrogène.

9. Composé pour son utilisation selon l'une quelconque des revendications précédentes, **caractérisé en ce que** R₆ représente un groupement alkyle tel qu'un groupement méthyle.

10. Composé pour son utilisation selon l'une quelconque des revendications précédentes, **caractérisé en ce que** R₇ représente un atome d'hydrogène.

11. Composé pour son utilisation selon l'une quelconque des revendications précédentes, **caractérisé en ce qu'**il est choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique Acide 2-(2-(4-Bromo-benzyl)-3-{hydroxy-[1-(1-isobutyryloxy-éthoxycarbonylamino)-éthyl]-phosphinoyl}-propionylamino)-propionique
Ester benzylique de l'acide 2-(2-Biphényl-4-ylméthyl-3-{hydroxy-[1-(ami no)-éthyl]-phosphinoyl}-propionylamino)-propioniq ue
Acide 2-(2-Biphényl-4-ylméthyl-3-{hydroxy-[1-(amino)-éthyl]-phosphinoyl}-propionylamino)-propionique
Ester éthylique de l'acide 2-(2-Biphényl-4-ylméthyl-3-{hydroxy-[1-(1amino)-éthyl]-phosphinoyl}-propionylamino)-propionique
Ester éthoxycarbonyloxy de l'acide 2-(2-Biphényl-4-ylméthyl-3-{hydroxy-[1-(amino)-éthyl]-phosphinoyl}-propionylamino)-propionique
Ester benzylique de l'acide 2-(2-(4-Bromo-benzyl)-3-{hydroxy-[1-(amino)-éthyl]-phosphinoyl}-propionylamino)-propionique
Acide 2-(2-(4-Bromo-benzyl)-3-{hydroxy-[1-(amino)-éthyl]-phosphinoyl}-propionylamino)-propionique
Ester éthylique de l'acide 2-(2-(4-Bromo-benzyl)-3-{hydroxy-[1-(amino)-éthyl]-phosphinoyl}-propionylamino)-propionique

12. Composé pour son utilisation selon l'une quelconque des revendications précédentes, formulé sous la forme d'une composition ophtalmique, en particulier de collyres, pommades ophtalmiques, gels ophtalmiques, ou inserts ophtalmiques.

13. Composé pour son utilisation selon la revendication 12, **caractérisé en ce que** la composition ophtalmique comprend de 0,01% à 3% en poids par volume dudit composé de formule (I), avantageusement de 0,1% à 1% en poids par volume.

14. Composé pour son utilisation selon la revendication 12 ou 13, **caractérisé en ce que** la composition ophtalmique est une solution aqueuse, ayant avantageusement un pH allant de 5,5 à 8.

15. Composé pour son utilisation selon la revendication 12 à 14, **caractérisé en ce que** la composition ophtalmique comprend en outre cyclodextrine, telle que l'hydroxypropyl béta-cyclodextrine ou bien encore la sulfobutyl ether béta-cyclodextrine, ou du sodium de polystyrène sulfonate.

## Patentansprüche

1. Verbindung, die der Formel (I) entspricht:
(I) R₁-NH-CH(R₂)-P(=O)(OH)-CH₂-C(R₃)(R₄)-CONH-C(R₅)(R₆)-COOR₇
wobei
R₁ steht für
- einen Wasserstoff,
- eine (Acyloxy)alkylcarbamat-Gruppe -C(=O)-O-C(R)(R')-OC(=O)-R", in der R et R' unabhängig voneinander für einen Wasserstoff, eine Alkylgruppe stehen, und R" für eine Alkylgruppe steht,
R₂ steht für:
eine gesättigte oder ungesättigte, geradkettige oder verzweigte Kohlenwasserstoffkette, die 1 bis 6 Kohlenstoffatome umfasst,
R₃ und R₄ unabhängig voneinander stehen für:
- einen Wasserstoff,
- eine Phenyl- oder Benzylgruppe, die gegebenenfalls am Phenylring substituiert ist mit:
-- 1 bis 5 Halogenatomen, insbesondere Fluor oder Brom,
-- einem OH-, SH-, OR"- oder SR"-Rest, wobei R" dieselbe Definition aufweist wie vorstehend,
-- einer Aminogruppe, die gegebenenfalls mit einer cyclischen oder geradkettigen aliphatischen Gruppe mit 1 bis 6 Kohlenstoffatomen mono- oder di-substituiert ist,
-- einer Trifluormethyl-Gruppe,
-- einer aromatischen oder heteroaromatischen Gruppe mit 5 oder 6 Atomen,
- eine heteroaromatische Gruppe mit 5 oder 6 Atomen, die 1 oder 2 Heteroatom(e) enthält, ausgewählt aus Sauerstoff, Stickstoff oder Schwefel, wobei die Schwefel- und Stickstoffatome in Form von S-Oxid oder N-Oxid oxidiert sein können,
- ein Methylen, das mit einem aromatischen oder gesättigten Heterocyclus mit 5 oder 6 Atomen substituiert ist, wobei das Heteroatom ein Sauerstoff, ein Stickstoff oder ein Schwefel ist, wobei die Stickstoff- und Schwefelatome in Form von N-Oxid oder S-Oxid oxidiert sein können,
R₃ und R₄ nicht gleichzeitig für ein Wasserstoffatom stehen,
R₅ und R₆ unabhängig voneinander stehen für
- ein Wasserstoffatom,
- eine gesättigte oder ungesättigte, geradkettige oder verzweigte Kohlenwasserstoffkette mit 1 bis 6 Kohlenstoffatomen,
R₇ steht für
- einen Wasserstoff,
- einen CH₂COOR'"- oder CH(CH₃)COOR" -Rest, wobei R''' steht für
-- eine gesättigte Kohlenwasserstoffkette mit 1 bis 6 Kohlenstoffatomen, die gegebenenfalls mit einer C₁ -bis C₃-Alkoxygruppe substituiert ist,
-- eine C₅ - bis C₈-Cycloalkylgruppe,
-- eine Phenyl, Benzyl, heteroaromatische oder alkylheteroaromatische Gruppe ,
- eine CH(R)O-C(O)OR'- oder CH(R)OC(O)R'-Gruppe, wobei R und R' dieselben Definitionen aufweisen wie vorstehend;
oder ein pharmazeutisch verträgliches Salz dieser Verbindungen für deren Verwendung in der Behandlung und/oder der Vorbeugung von Augenschmerzen.

2. Verbindung zu deren Verwendung nach Anspruch 1, um einem Augenschmerz, insbesondere dem Syndrom des trockenen Auges, vorzubeugen oder dasselbe zu lindern.

3. Verbindung zu deren Verwendung nach Anspruch 1 oder 2, um neuropathischen Schmerzen der Hornhaut, insbesondere der neuropathischen Hyperalgesie, vorzubeugen oder dieselben zu behandeln.

4. Verbindung zu deren Verwendung nach einem der vorstehenden Ansprüche, **dadurch gekennzeichnet, dass** R₁ für eine (Acyloxy)alkylcarbamat-Gruppe -C(=O)-O-C(R)(R')-OC(=O)-R" steht.

5. Verbindung zu deren Verwendung nach einem der vorstehenden Ansprüche, **dadurch gekennzeichnet, dass** R₁ für eine -C(=O)-O-CHMe-OC(=O)-CHMe₂-Gruppe steht.

6. Verbindung zu deren Verwendung nach einem der vorstehenden Ansprüche, **dadurch gekennzeichnet, dass** R₃ für ein Wasserstoffatom steht.

7. Verbindung zu deren Verwendung nach einem der vorstehenden Ansprüche, **dadurch gekennzeichnet, dass** R₄ für Benzylgruppe, die gegebenenfalls mit 1 bis 5 Halogenatom(en) wie etwa Fluor oder Brom substituiert ist, ein Phenyl oder eine 5- oder 6-gliedrige heteroaromatische Gruppe steht.

8. Verbindung zu deren Verwendung nach einem der vorstehenden Ansprüche, **dadurch gekennzeichnet, dass** R₅ für ein Wasserstoffatom steht.

9. Verbindung zu deren Verwendung nach einem der vorstehenden Ansprüche, **dadurch gekennzeichnet, dass** R₆ für eine Alkylgruppe, wie etwa eine Methylgruppe steht.

10. Verbindung zu deren Verwendung nach einem der vorstehenden Ansprüche, **dadurch gekennzeichnet, dass** R₇ für ein Wasserstoffatom steht.

11. Verbindung zu deren Verwendung nach einem der vorstehenden Ansprüche, **dadurch gekennzeichnet, dass** sie aus den folgenden Verbindungen ausgewählt ist:
2-(2-Biphenyl-4-ylmethyl-3-{hydroxy-[1-(1-isobutyryloxy-ethoxycarbonylamino)-ethyl]-phosphinoyl}-propionylamino)-propionsäure-benzylester,
2-(2-Biphenyl-4-ylmethyl-3-{hydroxy-[1-(1-isobutyryloxy-ethoxycarbonylamino)-ethyl]-phosphinoyl}-propionylamino)-propionsäure,
2-(2-Biphenyl-4-ylmethyl-3-{hydroxy-[1-(1-isobutyryloxy-ethoxycarbonylamino)-ethyl]-phosphinoyl}-propionylamino)-propionsäure-ethylester,
2-(2-Biphenyl-4-ylmethyl-3-{hydroxy-[1-(1-isobutyryloxy-ethoxycarbonylamino)-ethyl]-phosphinoyl}-propionylamino)-propionsäure-ethoxycarbonyloxyester,
2-(2-(4-Brombenzyl)-3-{hydroxy-[1-(1-isobutyryloxy-ethoxycarbonylamino)-ethyl]-phosphinoyl}-propionylamino)-propionsäure-benzylester,
2-(2-(4-Brombenzyl)-3-{hydroxy-[1-(1-isobutyryloxy-ethoxycarbonylamino)-ethyl]-phosphinoyl}-propionylamino)-propionsäure
2-(2-Biphenyl-4-ylmethyl-3-{hydroxy-[1-(amino)-ethyl]-phosphinoyl}-propionylamino)-propionsäure-benzylester,
2-(2-Biphenyl-4-ylmethyl-3-{hydroxy-[1-(amino)-ethyl]-phosphinoyl}-propionylamino)-propionsäure,
2-(2-Biphenyl-4-ylmethyl-3-{hydroxy-[1-(1amino)-ethyl]-phosphinoyl}-propionylamino)-propionsäure-ethylester,
2-(2-Biphenyl-4-ylmethyl-3-{hydroxy-[1-(amino)-ethyl]-phosphinoyl}-propionylamino)-propionsäure-ethoxycarbonyloxyester,
2-(2-(4-Brombenzyl)-3-{hydroxy-[1-(amino)-ethyl]-phosphinoyl}-propionylamino)-propionsäure-benzylester,
2-(2-(4-Brombenzyl)-3-{hydroxy-[1-(amino)-ethyl]-phosphinoyl}-propionylamino)-propionsäure,
2-(2-(4-Brombenzyl)-3-{hydroxy-[1-(amino)-ethyl]-phosphinoyl}-propionylamino)-propionsäure-ethylester.

12. Verbindung zu deren Verwendung nach einem der vorstehenden Ansprüche, die in der Form einer ophthalmischen Zusammensetzung, insbesondere von Augentropfen, ophthalmischen Salben, ophthalmischen Gelen oder ophthalmischen Einlagen formuliert ist.

13. Verbindung zu deren Verwendung nach Anspruch 12, **dadurch gekennzeichnet, dass** die ophthalmische Zusammensetzung 0,01 Gewichts-% bis 3 Gewichts-% pro Volumen der Verbindung der Formel (I), vorteilhafterweise 0,1 Gewichts-% bis 1 Gewichts-% pro Volumen umfasst.

14. Verbindung zu deren Verwendung nach Anspruch 12 oder 13, **dadurch gekennzeichnet, dass** die ophthalmische Zusammensetzung eine wässrige Lösung ist, die vorteilhafterweise einen pH-Wert im Bereich von 5,5 bis 8 aufweist.

15. Verbindung zu deren Verwendung nach Anspruch 12 bis 14, **dadurch gekennzeichnet, dass** die ophthalmische Zusammensetzung weiter Cyclodextrin, wie etwa Hydroxypropyl-beta-cyclodextrin oder auch Sulfobutylether-beta-cyclodextrin, oder Natriumpolystyrolsulfonat umfasst.

## Claims

1. Compounds having the formula (I):
(I) R₁-NH-CH(R₂)-P(=O)(OH)-CH₂-C(R₃)(R₄)-CONH-C(R₅)(R₆)-COOR₇
Wherein:
R₁ is
- a hydrogen
- an (acyloxy)alkyl carbamate group -C(=O)-O-C(R)(R')-OC(=O)-R" wherein R and R' are each independently a hydrogen, an alkyl group and R" is an alkyl group.
R₂ is:
- a linear or branched, saturated or unsaturated hydrocarbon chain having from 1 to 6 carbon atoms
R₃ and R₄ are each independently:
- a hydrogen
- a phenyl or benzyl group, optionally substituted on the phenyl ring by:
*1 to 5 halogen atoms, particularly fluorine or bromine.
*an OH, SH, OR" or SR" radical, R" having the same definition as above.
*an amino group optionally mono- or di-substituted by a cyclic or linear aliphatic group having from 1 to 6 carbon atoms.
*a trifluoromethyl group
*an aromatic or heteroaromatic group having 5 or 6 atoms
- a heteroaromatic group having 5 or 6 atoms, containing 1 or 2 heteroatom(s) selected from oxygen, nitrogen or sulphur, wherein the sulphur and nitrogen atoms may be oxidized in S-oxide or N-oxide form.
- a methylene substituted by an aromatic or saturated heterocycle having 5 or 6 atoms, the heteroatom being an oxygen, a nitrogen or a sulphur, wherein the nitrogen and sulphur atoms may be oxidized in N-oxide or S-oxide form.
R₃ and R₄ are not simultaneously a hydrogen atom
R₅ and R₆ are each independently
- a hydrogen atom
- a linear or branched, saturated or unsaturated hydrocarbon chain having from 1 to 6 carbon atoms
R₇ is
- a hydrogen
- a CH₂COOR'" or CH(CH₃)COOR'" radical, R'" being
*a saturated hydrocarbon chain having from 1 to 6 carbon atoms, optionally substituted by a C₁ to C₃ alkoxy group,
*a C₅ to C₈ cycloalkyl group
*a phenyl, benzyl, heteroaromatic or alkylheteroaromatic group.
- a CH(R)O-C(O)OR' or CH(R)OC(O)R' group wherein R and R' have the same definitions as above;
or a pharmaceutically acceptable salt of said compounds for use in the treatment and/or prevention of eye pain.

2. The compound for use according to claim 1, for preventing or relieving eye pain, in particular dry eye syndrome.

3. The compound for use according to claim 1 or 2, for preventing or treating corneal neuropathic pain, in particular neuropathic hyperalgesia.

4. The compound for use according to any one of the preceding claims, **characterized in that** R₁ is an (acyloxy)alkyl carbamate group -C(=O)-O-C(R)(R')-OC(=O)-R".

5. The compound for use according to any one of the preceding claims, **characterized in that** R₁ is a -C(=O)-O-CHMe-OC(=O)-CHMe₂ group.

6. The compound for use according to any one of the preceding claims, **characterized in that** R₃ is a hydrogen atom.

7. The compound for use according to any one of the preceding claims, **characterized in that** R₄ is benzyl group optionally substituted by 1 to 5 halogen atom(s) such as fluorine or bromine, a phenyl or a 5- or 6-member heteroaromatic group.

8. The compound for use according to any one of the preceding claims, **characterized in that** R₅ is a hydrogen atom.

9. The compound for use according to any one of the preceding claims, **characterized in that** R₆ is an alkyl group such as a methyl group.

10. The compound for use according to any one of the preceding claims, **characterized in that** R₇ is a hydrogen atom.

11. The compound for use according to any one of the preceding claims, **characterized in that** it is selected from the following compounds:
2-(2-Biphenyl-4-ylmethyl-3-{hydroxy-[1-(1-isobutyryloxy-ethoxycarbonylamino)-ethyl]-phosphinoyl}-propionylamino)-propionic acid benzyl ester
2-(2-Biphenyl-4-ylmethyl-3-{hydroxy-[1-(1-isobutyryloxy-ethoxycarbonylamino)-ethyl]-phosphinoyl}-propionylamino)-propionic acid
2-(2-Biphenyl-4-ylmethyl-3-{hydroxy-[1-(1-isobutyryloxy-ethoxycarbonylamino)-ethyl]-phosphinoyl}-propionylamino)-propionic acid ethyl ester
2-(2-Biphenyl-4-ylmethyl-3-{hydroxy-[1-(1-isobutyryloxy-ethoxycarbonylamino)-ethyl]-phosphinoyl}-propionylamino)-propionic acid ethoxycarbonyloxy ester
2-(2-(4-Bromo-benzyl)-3-{hydroxy-[1-(1-isobutyryloxy-ethoxycarbonylamino)-ethyl]-phosphinoyl}-propionylamino)-propionic acid benzyl ester
2-(2-(4-Bromo-benzyl)-3-{hydroxy-[1-(1-isobutyryloxy-ethoxycarbonylamino)-ethyl]-phosphinoyl}-propionylamino)-propionic acid
2-(2-Biphenyl-4-ylmethyl-3-{hydroxy-[1-(amino)-ethyl]-phosphinoyl}-propionylamino)-propionic acid benzyl ester
2-(2-Biphenyl-4-ylmethyl-3-{hydroxy-[1-(amino)-ethyl]-phosphinoyl}-propionylamino)-propionic acid
2-(2-Biphenyl-4-ylmethyl-3-{hydroxy-[1-(1amino)-ethyl]-phosphinoyl}-propionylamino)-propionic acid ethyl ester
2-(2-Biphenyl-4-ylmethyl-3-{hydroxy-[1-(amino)-ethyl]-phosphinoyl}-propionylamino)-propionic acid ethoxycarbonyloxy ester
2-(2-(4-Bromo-benzyl)-3-{hydroxy-[1-(amino)-ethyl]-phosphinoyl}-propionylamino)-propionic acid benzyl ester
2-(2-(4-Bromo-benzyl)-3-{hydroxy-[1-(amino)-ethyl]-phosphinoyl}-propionylamino)-propionic acid
2-(2-(4-Bromo-benzyl)-3-{hydroxy-[1-(amino)-ethyl]-phosphinoyl}-propionylamino)-propionic acid ethyl ester

12. The compound for use according to any one of the preceding claims, formulated as an ophthalmic composition, in particular as eye drops, ophthalmic ointments, ophthalmic gels, or ophthalmic inserts.

13. The compound for use according to claim 12, **characterized in that** the ophthalmic composition comprises from 0.01% to 3% (weight/volume) of said compound of formula (I), advantageously from 0.1% to 1% (weight/volume).

14. The compound for use according to claim 12 or 13, **characterized in that** the ophthalmic composition is an aqueous solution, advantageously having a pH in the range from 5.5 to 8.

15. The compound for use according to claim 12 to 14, **characterized in that** the ophthalmic composition further comprises cyclodextrin, such as hydroxypropyl beta-cyclodextrin or sulphobutyl ether beta-cyclodextrin, or sodium polystyrene sulphonate.
